# EUROPEAN PATENT SPECIFICATION

(11) **EP 1 049 686 B1**
(45) Date of publication and mention of the grant of the patent: **02.11.2005**
(21) Application number: 98954684.1
(22) Date of filing: 19.11.1998
(51) Int. Cl.: C07D 305/14, C07D 263/06

(54) **INTERMEDIATES AND METHODS USEFUL IN THE SEMISYNTHESIS OF PACLITAXEL AND ANALOGS**
ZWISCHENPRODUKT UND VERFAHREN ZUR TEILSYNTHESE VON PACLITAXEL UND DESSEN ANALOGEN
INTERMEDIAIRES ET PROCEDES UTILES DANS LA SEMISYNTHESE DE PACLITAXEL ET DE SES ANALOGUES

(30) Priority: 21.11.1997 US 975804
(43) Date of publication of application: 08.11.2000
(73) Proprietor: INDENA S.p.A., 20139 Milano (IT)
(72) Inventor: BOMBARDELLI, Ezio, I-20139 Milano (IT)
(74) Representative: Naylor, Kathryn M.
(86) International application number: PCT/IB1998/001912
(87) International publication number: WO 1999/026939

(56) References cited:
- EP-A- 0 336 840
- EP-A- 0 617 034
- EP-A- 0 735 036
- WO-A-93/06094
- WO-A-94/07879
- WO-A-98/08832
- US-A- 5 476 954
- US-E- R E34 277

## Description

### TECHNICAL FIELD

The present invention relates to semisynthesis of paclitaxel and its analogs using new intermediates which are derivatives of 10-deacetyl-baccatine III, as well as to a method for preparing these derivatives. These novel derivatives have carbonate substituents in the 7 position, such as t-butoxy-carbonate.

### BACKGROUND ART

Paclitaxel, a well known potent antitumor compound having a broad spectrum of antitumor activities, has the following structure of formula (I):

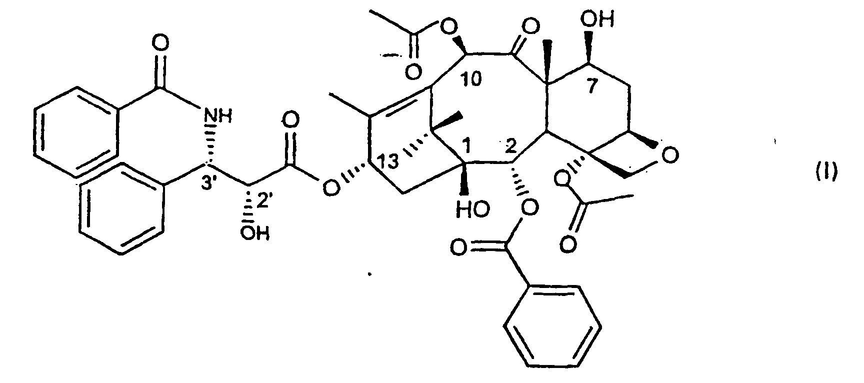

Commercial pharmaceutical products containing this compound are available, e.g., for treating ovarian and breast cancer in women. For these reasons, greater and greater supplies of this compound are required each year. Paclitaxel and baccatine III are extracted with difficulty and in general in low yields from the trunk barks of different Taxus species. Thus, alternative sources of this compound are necessary.

Several synthetic methods have been reported both in scientific and patent literature. U.S. patent RE-34,277 (a reissue of U.S. patent 4,924,011) discloses the semisynthesis of paclitaxel using a 10-deacetyl-baccatine III derivative which is protected in the 7 position with a tri-alkyl-silyl group which is specifically shown as a tri-ethyl-silyl ("TES") group and which is also protected in the 10 position with an acetyl group. This baccatine III derivative is allowed to react with a (2R,3S)-N-benzoyl-2-O-(1-ethoxyethyl)-3-phenyl-isoserine compound before removal of the protecting groups to obtain the paclitaxel.

In PCT application WO-93/06094, paclitaxel was prepared by reacting a side chain precursor of a β-lactam compound with 7-O-TES-baccatine III derivative to provide a 7-TES-baccatin III reaction product. After a mild acidic post-reaction treatment, paclitaxel was obtained.

In U.S. patent 5,476,954, the synthesis of paclitaxel was conducted starting from a protected 10-dsacetyl-baccatine III derivative that contained a 2,2,2-tri-chloroethoxy-carbonyl ("TROC") protective group in both the 7 and 10 positions of the derivative.

EP 0 617 034 discloses a process to form 2-debenzoyl-2-acyl taxol derivatives. In the disclosed process, C-13-substituted, 2-benzoyl taxol analogues in which the C-7 and C-10 hydroxyl groups are protected with various protecting groups including ^{t}BOC, are selectively deprotected and re-acylated at the C-2 position to form the 2-debenzoyl-2-acyl analogues.

WO 94/07879 discloses a method for preparing taxane derivatives by esterification of a protected baccatine III or 10-deacetylbaccatine III using an oxazolidine acid. The baccatine starting materials are preferably protected at the C7-position with a trichloroothyl carbonyl or a trichloropropyl carbonyl radical.

EP 0735 036 discloses a method for the preparation of taxanes using substituted oxazolidines. In the disclosed procedure, C-13-substituted taxanes are prepared by reacting the oxazolidines with a taxane moiety which is protected using various protecting groups. The C-7 hydroxyl group of the taxane moiety is preferably protected with trialkylsilyl.

It is well known that the key step in the semisynthesis of paclitaxel is to selectively protect the 7 position with a leaving group that can be easily removed. This is because the hydroxy group in that position of the taxane structure is much more reactive than those in position 10 or 13, and the paclitaxel product to be synthesized needs to have a hydroxy group in that position. Until now, however, the most useful protecting group was considered to be TES. The derivatization yield of 10-deacetyl-baccatine III with TES is typically about 85% when 20 moles of the reagent are used. The acetylation step, using 5 equivalents of acetylchloride, provides about 85% of 7-TES-baccatine III. as per the teachings of PCT application WO-93/06094 and its U.S. equavalent documents such as U.S. Patent 5,574,156.

In view of the importance of paclitaxel, however, new and improved methods for its production are desirable.

The present invention provides such improved syntheses of paclitaxel and its analogues primarily using new derivatives of 10-deacetyl-baccatin III as intermediates.

### SUMMARY OF THE INVENTION

The present invention relates to intermediates for use in the semisynthesis of paclitaxel or a paclitaxel analogue, comprising a compound of the structure: 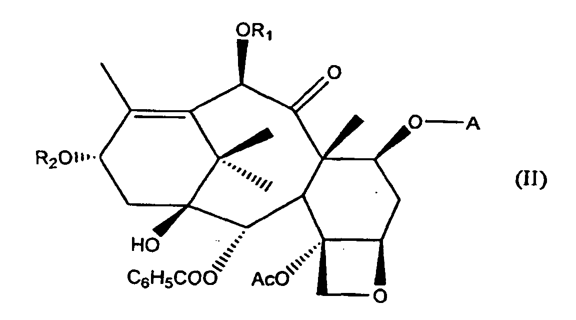 wherein:
A is 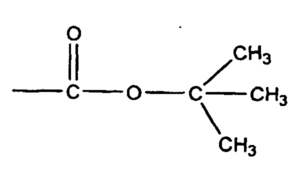
R₁ is a hydroxy-protecting group or a hydrogen atom, and
R₂ is a hydrogen atom.

The invention further provides intermediates for use in the semisynthesis of paclitaxel or a paclitaxel analogue, comprising a compound of formula (III): 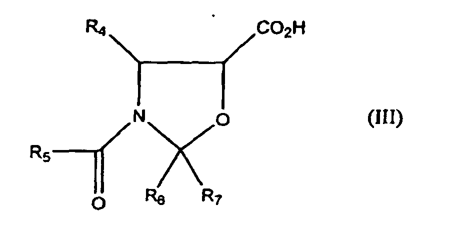 wherein R₄ is an aryl group or a straight or branched chain alkyl or alkenyl group having 1-5 carbon atoms: and R₅ is R₄ or a t-butoxy group, and each of R₆ and R₇ is a halogenated methyl group.

In its process aspects, the invention provides a process for producing paclitaxel or docetaxel comprising:
(i) forming an intermediate compound by reacting 10-deacetyl-baccatine III with t-butoxy-pyrocarbonate to obtain 7-t-butoxy-carbonyl-10-deacetyl baccatine III;
(ii) acetylating the 10-position of the 7-t-butoxy-carbonyl-10-deacetyl baccatine III to obtain 7-t-butoxy-carbonyl-baccatine III;
(iii) introducing the group 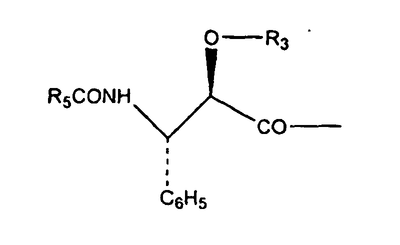 wherein R₃ is a hydrogen atom and R₅ is phenyl or t-butoxy; in a position 13 of 7-t-butoxy-carbonyl baccatine III by reacting the protected baccatine III with an oxazolidine derivative of formula (III): 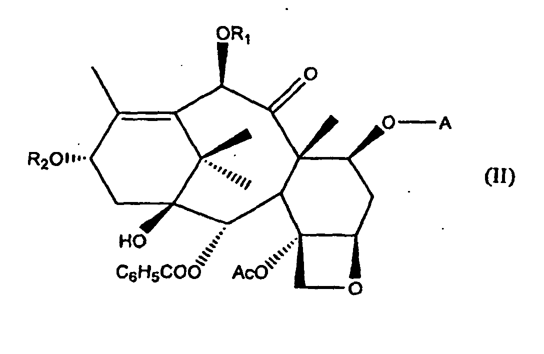 wherein R₄ is phenyl and R₅ is phenyl or a t-butoxy group, and each of R₆ and R₇ is a halogenated methyl group, and selectively removing the A group in mild acidic conditions using a mineral or organic acid.

Examples of hydroxy-protecting groups include C₁₋₄ carboxylic acid acyl groups, for example acetyl, trialkylsilyl groups wherein each alkyl group contains 1 to 3 carbon atoms and the group A defined above, e.g. a t-butoxycarbonyl group of formula

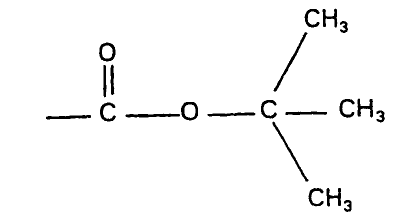

Preferably, therefore R₁ is a C₁₋₄ carboxylic acid acyl group, for example acetyl, a trialkylsilyl group wherein each alkyl group contains 1 to 3 carbon atoms or the group A defined above, e.g. a t-butoxycarbonyl group

R₂ may be any of these groups, but because the 13-hydroxy group is less reactive, protection is not essential, so R₂ can conveniently be hydrogen.

During the semisynthesis of paclitaxel, an N-benzoyl (2R, 3S)-3-phenylisoserine group is introduced at the 13-position of an appropriately protected derivative of baccatine III. The resulting protected derivatives of paclitaxel have the general formula (IIa) 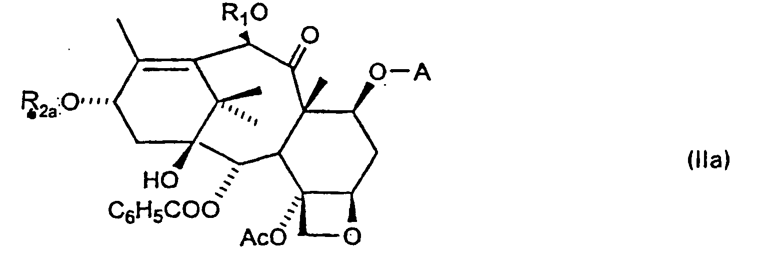 wherein A is 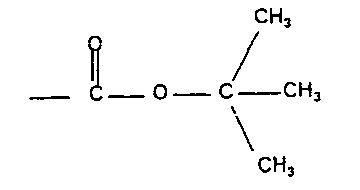 R₁ is a hydroxy-protecting group or a hydrogen atom; and
R₂ₐ is a (2R,3S)-3-phenylisoserine derivative having the structure: 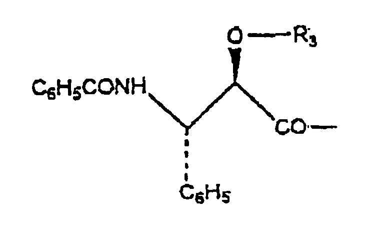 where R₃ is a hydroxy-protecting group, such as A (as defined above), a methoxy methyl, 1-ethoxyethyl, benzyloxymethyl, β-trialkylsilylethoxy)methyl where each alkyl group contains 1 to 3 carbon atoms, tetrahydropyranyl or 2,2,2-trichloroethoxycarbonyl group; or a hydrogen atom.

The invention further relates to a process for producing paclitaxel by the steps of forming the intermediate compound of formula (IIa) and removing the A and R₃ groups to form paclitaxel.

For the preparation of compounds of formula II and IIa where R₁ is acetyl the process of the invention preferably comprises forming the intermediate compound II by reacting 10-deacetylbaccatine III with a reagent capable of introducing a t-butoxycarbonyl group, for example t-butoxy-pyrocarbonate to obtain 7-t-butoxycarbonyl-10-deacetyl baccatine III. The thus obtained 7-t-butoxycarbonyl-10-deacetyl baccatine III may then be acetylated to obtain 7-t-butoxycarbonyl-baccatine III.

10-Deacetylbaceatine III is highly insoluble in most common solvents and accordingly the choice of solvent is important in order to ensure that the reaction in which the t-butoxycarbonyl group is introduced proceeds at an acceptable rate and in high yields. Thus, for example, 10-deacetyl is highly insoluble in methylene dichloride. If methylene dichloride is used as a solvent for the reaction of 10-deacetylbaccatine with t-butoxypyrocarbonate (see, e.g. Example 1 below), the reaction proceeds at a reasonably fast rate on a small scale, but when operated on a large scale, the reaction can be unacceptably slow.

On the other hand, if a polar aprotic nitrogen-containing solvent such as pyridine is used for carrying out the reaction (see Example 8 below) the reaction proceeds more rapidly, but the yield can be lower as a result of formation of 7,10-di(t-butoxycarbonyl) baccatin as a by-product.

Although there may be a minor penalty in terms of lower yield, the use of pyridine as a solvent has advantages on the industrial scale in view of the higher rate of the reaction. Also it is possible to carry out the next step in the process, i.e. the introduction of an acetyl group in the 10-position, without separating or purifying the desired 7-t-butoxycarbonyl 10-deacetylbaccatin. I.e. the reaction in which the t-butoxycarbonyl group is introduced and the acetylation reaction can be carried out in one pot.

Further, if the starting material is in the amorphous (anhydrous) form the reaction proceeds more rapidly than if the starting material is in the form of the crystalline hemihydrate.

The hydroxy group in position 13 of the 7-t-butoxy- carbonyl baccatine III may then be converted to a (2R,3S)-3-phenylisoserine group having the structure 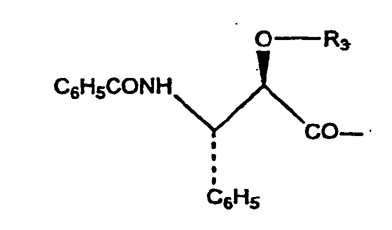 wherein R₃ is hydrogen by reacting the 7-t-butoxy-carbonyl-baccatine III with an oxazolidine derivative of formula (III): 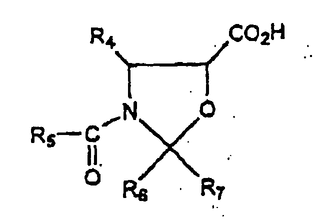 wherein R₄ is an aryl group or a straight or branched chain alkyl or alkenyl group having 1-5 carbon atoms; and R₅ is R₄ or a t-butoxy group, and each of R₆ and R₇ is a halogenated methyl group. Advantageously, an excess of the oxazolidine derivative is used relative to the 7-t-butoxy-carbonyl-baccatine III compound.

The oxazolidine derivatives of formula (III) 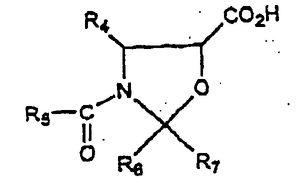 where R₄, R₅, R₆ and R₇ are as defined above form a further aspect of the present invention. Preferably, R₄ is phenyl, R₅ is phenyl or a t-butoxy group, and each of R₆ and R₇ is a ClCH₂-, BrCH₂- or F₃C- group.

The oxazolidine derivatives of Formula III may be prepared by reacting a compound of Formula IV with a ketone of formula R₆R₇CO (V)

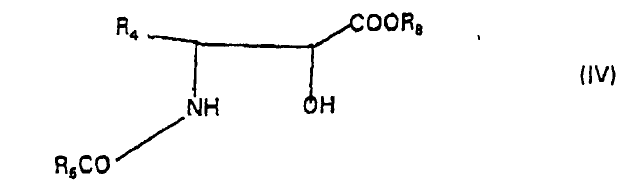

In formula IV and V R₄, R₅, R₆ and R₇ are as defined above, and R₈ is a residue of an alcohol R₈ OH in whic R₆ is, e.g. a C₁₋₄ alkyl group, e.g. methyl.

### DETAILED DESCRIPTION OF THE INVENTION

The present invention provides novel methods for the semisynthesis of paclitaxel of the general formula (I) given above through the use of the new intermediates of formulae (II) and (IIa). These new intermediates are versatile intermediates which also can be used for the semisynthesis of docetaxel and other analogs of paclitaxel. The process for their preparation is also described.

It has been found, surprisingly, that protecting the hydroxy group at position 7 of 10-deacetylbaccatine III or similar taxane derivatives with the same basic structure, with certain carbonate compounds provides enhancements in the preparation of paclitaxel from such derivatives.

A preferred protective group is t-butoxy-pyrocarbonate (BOC): 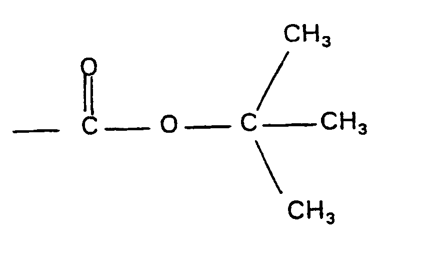 This protective group can be substituted in position 7 and, if desired, as well as in position 10.

As position 10 is not as reactive as position 7, a number of other protective groups can be used in position 10. In particular, the group -OR, can be used, where R₁ is a hydroxy-protecting group or a hydrogen atom. Any of a wide variety of hydroxy-protecting groups can be used, including the carbonate groups described above for A, the G₁ groups of the compounds of formula III of U.S. Patents 5,578,739 or 5,621,121, the R₂ groups of the compounds of formula III of U.S. patent 5,476,954, or the R₃ substituents of the compounds of formula IV of U.S. patent Re. 34,277.

It is possible to obtain almost quantitative yields of the 7-BOC-10-deacetylbaccatine III derivative from 10-deacetylbaccatine III. The BOC protecting group is easily and selectively removed in very mild acidic conditions using a catalytic amount of mineral or organic acids, preferably formic or F₃C-COOH.

The synthesis of 7-BOC-10-deacetylbaccatine III or its analog may be performed In chlorinated solvents, preferably in methylene chloride using dimethylformamide as a co-solvent. 1 Mole of 10-deacetyl-baccatine III or the chosen taxane analog may be reacted with 1.2 to 2.5 equivalents t-terbutoxy-pyrocarbonate in the presence of 1.2 equivalents of ethyldiisopropylamine and a catalytic amount of 4-dimethylaminopyridine. Under these conditions, it is possible to obtain in almost quantitative yields the 7-BOC-derivative. This compound can be converted into 7-BOC-10-acetyl derivative using acetyl chloride, bromide or diketene as shown in the examples.

These derivatives can then be converted to biologically active compounds by esterifying the hydroxy group at the 13 position with an oxazolidine derivative of formula (III): 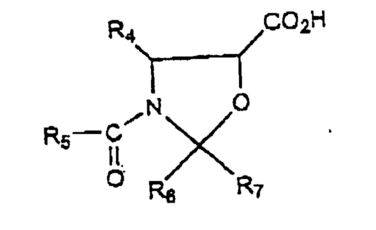 wherein R₄ is an aryl group or a straight or branched chain alkyl or alkenyl group having 1-5 carbon atoms; and R₅ is R₄ or a t-butoxy group, and each of R₆ and R₇ is a halogenated methyl group.

The reaction is generally performed in aprotic solvents, preferably benzene, toluene, xylene, chlorobenzene or ethylbenzene, preferably in the presence of a condensing agent such as dicyclohexylcarbodiimide (DCC) and a catalytic amount of a base such as dialkylamino pyridine, preferably 4-dimethylaminopyridine at temperatures ranging from about 50°C to 100°C, and preferably 70°C.

Preferably, to obtain the desired compounds, 4 Moles of condensing agent and 1.5 Mole of the oxazolidine derivative are used for 1 Mole of protected taxane. After elimination of the reaction byproducts and the solvent, the 13-ester may be isolated in crude form. This compound may then be treated in methanol with a catalytic amount of anhydrous HCl at room temperature or at temperatures ranging from about 5°C to 10°C, and preferably at 0°C, with concentrated formic acid (98%) until complete deprotection of the BOC group at the 7 position and of the protective group R₃ of the side chain at position 13 is achieved. After treatment of the reaction mixture with brine, the taxane derivative may be extracted with a solvent that is non-miscible with water, and preferably with ethylacetate. After distillation of the extraction solvent, the taxane derivatives may directly be crystallized with suitable solvents or subjected to chromatographic process using silica-gel and as eluting solvents, a mixture preferably constituted by hexane/ethylacetate in a suitable ratio.

Alternatively, paclitaxel and its analogs can be prepared by esterifying the protected baccatine with a phenylisoserine chain esterified at 2 position with BOC. The reaction conditions described above for the reaction using a oxazolidine derivative may be used.

The hydroxy group at position 13 can be esterified in a number of other ways as disclosed, e.g., in U.S. patents 5,578,739, 5,574,156, 5,621,121, 5,476,954, 5,470,866, 4,857,653, 4,814,470, and Re. 34,277, and in European Patent Application 0,525,589-A1. To the extent necessary to understand the present invention, all patents cited in the detailed description of this specification are expressly incorporated by reference herein.

### EXAMPLES

The examples below are reported, without implied limitation, to show how the invention can be put in practice.

### Example 1: Synthesis of 7-BOC-10-deacetylbaccatine III.

A 500 mg sample of 10-deacetylbaccatine III (0.92 mMol) was suspended in CH₂Cl₂ (5 mL) and ethyldiisopropylammine (1.10 mMol, 1.2 Equiv.), t-butoxypyrocarbonate (240 mg, 1.10 mMol, 1.2 Equiv.) and DMAP (4-dimethylaminopyridine, 20 mg) were added.

The reaction was stirred 48 h at room temperature and then additioned with the same quantity of reagents and allowed to stay under stirring per other 48 h. The reaction was worked up by dilution with CH₂Cl₂ washing with HCl and brine. After drying, 580 mg of 7-Boc- 10-deacetylbaccatine III were obtained having the following characteristics: mp 148°C and 162°C; 1H-NMR 200 Mhz, CDCl3, TMS as internal standard; Bz 8.10, br d, J 8; Bz 7.70, br t J 8; Bz 7.55, br t J 8; H2, 5.64 d J 7; H10, 5.54, s; H7, 5.36, dd, J 11.0, 8.0; H5, 4.95, d J 8; H13, 4.91, br t, J7.5; H₂Oa, 4.32 d, J 8.0; H₂Ob 4.26, d, J 8.0; H3, 4.09 d, J 8.8; Ac. 2.29 s; H 18 2.09 s; H 19 1.83 s; Boc 1.46 s; H16 1.34 s; H17 1.20 s; IR (KBr) 3480 (OH), 1740 (br, C = O), 1603, 1371, 1275, 1259, 1158, 1092, 712.

### Example 2: Synthesis of 7-BOC-10-deacetylbaccatine III

A 500 mg sample of 10-deacetylbaccatine III (0.92 mmol) was solubilized in 1 ml of dimethylformamide and diluted with 4 ml of CH₂Cl₂. The reagents and the reaction conditions are the same of Example I.

### Example 3: Synthesis of 7-BOC-baccatine III.

644 mg (1 mMol) of 7-Boc-10-deacetylbaccatine III prepared according to example 1 or 2 were dissolved in 5 mL of pyridine and at 0°C under stirring 1.2 g of acetylchloride were added (15 mmol) in 15 h. When the reaction is finished the solution is diluted with CH₂Cl₂ under stirring and washed with 60 mL of H₂O. The organic phase is washed several times with H₂O and diluted HCl until the elimination of pyridine. The solvent dried on Na₂SO₄ is evaporated under vacuum and the residue crystallized from hexane/acetone. 660 mg of 7-Boc-baccatine III were obtained having the following characteristics: mp 190-97 °C. 1H-NMR 200 Mhz, CDCl3, TMS as internal standard; Bz 8.10 br d, J 8; Bz 7.70 br t, J 8; Bz 7.55, br t J 8; H2, 5.64 d, J 7; H10, 5.52 s; H7, 5.44 dd, J 10.3, 7.0; H5, 4.98, d, J 7.9; H 13, 4.50 br t; H₂Oa, 4.32 d, J 8.0; H₂Ob 4.22 d, J 8.0; H3, 4.02 d, J 6.7; Ac. 2.30 s; H18 2.19 s; Ac. 2.16 s; H19 1.80 s; Boc 1.48 s; H16 1.17 s; H17 1.07 s.

### Example 4: Synthesis of paclitaxel.

1.65 gr of (4S,5R)-2,2 -di(chloromethyl)-4-phenyl-N-benzoyl-5-oxazolidine acid were allowed to react in toluene with 0.69 gr of 7-Boc-baccatine III in the presence of 1.1 Equival. of DCC and 60 mg of 4-dimethylaminopyridine. The reaction mixture was maintained at 60°C for 12h under stirring in Argon atmosphere.

At the end of the reaction (TLC) the reaction mixture was filtered form insoluble byproducts and the solvent washed with H₂O and distilled under vacuum. The residue is solubilized in 10 mL of conc. formic acid at 0°C and kept in this condition for 2h. The reaction mixture was diluted with 100 mL of H₂O and cloudy solution extracted three times with 50 mL CH₂Cl₂. The organic phase was washed with a solution of NaHCO3 and then with H₂O. The organic phase after drying on Na₂SO₄ is concentrated under vacuum. The residue was crystallized from ethanol/water and 0.81 gr of paclitaxel having the well known characteristics which have been reported in the literature was obtained.

### Example 5: Reaction of 10-deacetylbaccatin III with Boc-pyrocarbonate

A 500 mg sample of 10-deacetylbaccatin III (0.92 mMol) was suspended in CH₂Cl₂ (5 mL) and ethyldiisopropylamine (190 µL, 1.10 mMol, 1.2 mol. Equiv.), BOC-pyrocarbonate (240 mg, 1.10 mmol, 1.2 mol. Equiv) and DMAP (4-dimethylaminopyridine, 20 mg) were added. The reaction was stirred 48 h at room temp, and then further BOC-pyrocarbonate (240 mg, 1.10 mMol, overall 2.4 mol equiv.) and ethyldiisopropylamine (190 µL, 1.10 mMol, overall 2.4 mol , equiv.) were added. After stirring for an additional 120 hours, the reaction was worked up by dilution with CH₂Cl₂, washing with HCl and brine. After drying the residue was purified by column chromatography (ca. 5 g silica gel). Elution with hexane-EtOAc 6:4 gave 327 mg BOC DAB (yield: 55% conversion: 92%) Elution with ETOAc gave 195 mg recovered DAB.

### Example 6 - Reaction of 10-deacetylbaccatin III With BOC-ON

To a solution of 10-deacetylbaccatin III (400 mg, 0.73 mmol) in pyridine (3 mL), BOC-ON [=2-(tert-butoxycarbonyloxiymino)2-phenylacetonitrite] - (543 mg, 2.19 mmol, 3 mol. equiv.) and 4-dimethylaminopyridine (90 mg, 0.73 mmol, 1 mol.equiv) were added.

The reaction was followed by TLC (hexane-EtOAc 4:6, Rf starting material: 0.1; Rf 7-BOC derivative 0.50; Rf 7,10-diBOC derivative: 0.56). After stirring at room temp. for 10 days, the reaction was worked up by dilution with water and extraction with chloroform. After washing with sat. citric acid, sat. NaHCO₃ and brine, the solution was dried (MgSO₄) and evaporated, to afford a semi-solid residue (1.07 g). The latter, when analyzed by ¹H NMR spectroscopy (200 MHz), turned out to contain the 7-BOC and the 7,10-diBOC derivatives in a 85:15 ratio. Purification by column chromatography (hexane-EtOAc 6:4) afforded 265 mg of 7-BOC -10-deacetylbaccatin III (yield: 56%).

### Analysis - 7 BOC-10-deactylbaccatin III

White powder, mp 162 °C;
IR (KBr): 3480, 1740, 1603, 1371, 1275, 1259, 1158, 1092, 712
CI-MS: 645 (M + H), C₃₄H₄₄O₁₂
¹H NMR (200 MHz, CDCl₃) : 8.10 (br d, J = 8.0 Hz, Bz); 7.70 (br t, J = 8.0 Hz, Bz).
7.55 (br t, J = 8.0 Hz, Bz), 5.64 (d, J = 7.0 Hz, H-2), 5.54 (s, H-10), 5.36 (dd, J = 11.0, 8.0 Hz, H-7), 4.95 (d, J = 8.0 Hz, H-5), 4.91 (br t, J = 7.5 Hz, H-13), 4.32
(d, J = 8.0 Hz, H-20a), 4.26 (d, J = 8.0 Hz, H-20b), 4.09 (d, J = 8.0 Hz, H-3), 2.29
(s, OAc), 2.09 (br s, H-18), 1.83 (s, H-19), 1.46 (s, BOC), 1.34 (s, H-16), 1.20 (s, H-17).

### Analysis - 7-BOC-baccatin III

White powder, mp 197 °C;
IR (KBr): 3580, 3497,1750,1724,1713,1273,1240,1070,980.
M.W.:686, C₃₆H₄₆O₁₃
¹H NMR (200 MHz, CDCl₃): 8.10 (br d, J = 8.0 Hz, Bz );7.70 (br t, J = 8.0 Hz, Bz).
7.55 (br t, J = 8.0 Hz, Bz), 6.52 (s, H-10), 5.64 (d, J = 7.0 Hz, H-2), 5.41 (dd, J = 11.0, 8.0 Hz, H-7), 4.98 (d, J = 8.0 Hz, H-5), 4.90 (br t, J = 7.5 Hz, H-2), 4.32 (d, J = 8.0 Hz, H-20a), 4.22 (d, J = 8.0 Hz, H-20b), 4.02 (d,J = 7.0 Hz, H-3), 2.30 (s, OAc), 2.19 (br s, H-18), 2.16 (s, OAc), 1.80 (s, H-19), 1.48 (s, BOC), 1.17 (s, H-16), 1.07 (s, H-17).

### Example 7 - Preparation of Dichlorooxazolidine Derivative IIIa (Methyl Ester of Compound of Formula III with R₄ = R₅ = phenyl; R₆ = R₇ = Cl)

500 mg of N-benzoyl-phenyl-isoserine methylester in 30ml of tetrahydrofuran/benzene 1:1 mixture was allowed to react with 1g of dichloroacetone and 50 mg of PTSA (pyridinium p-toluenesulfonate) in the presence of a molecular sieve (3Å). The reaction mixture was heated and refluxed for 2 days. At the end of the reaction, the residue was washed with hexane in order to eliminate the excess of dichloroacetone.

The residue in 20ml of MeOH was mixed with 220 mg of K₂CO₃ in 20ml of H₂O. After 2 hours, methanol was evaporated under vacuum and the aqueous phase was acidified with a 5% solution of KHSO₄ and then extracted with ethylacetate.

The obtained acid was used then used directly for the esterification of 7-Boc-10 acetylbaccatine III (see Example 4).

### Example 8 - Reaction of 10-Deacetylbaccatin III with Boc-Pyrocarbonate

BOC₂O (800 mg, 37 mmol, 2 mol. equiv.) and DMAP (220 mg, 18.5 mMol, 1 mol. equiv.) were added to a solution of 10-deacetylbaccatin III (1.0 g, 18.5 mMol) in pyridine (15 mL), . The reaction was followed by TLC (hexane-EtOAc f:6, Rf st.m = 0.10; Rf7-boc-derivative 0.50; Rf 7,10-diBOC derivative: 0.56).

After stirring (16 h at room temp.), the reaction mixture was cooled to 0°C, and AcCl (261 µL, 37 mmol, 2 mol. equiv.) was added. The reaction was followed by TLC (1,2-dichloroethane-EtOH 96:4 x 4).

After stirring at 0°C for 5 h, two further equivalents of AcCl were added, and stirring is continued at 0° for a further 2 hours. The reaction mixture was then worked up by addition of water (*ca* 150 mL) to the reaction flask. After 30 min. water was decanted from the reaction flask, and the sticky precipitate on the walls of the was taken up in EtOAc (30 mL), washed with dil HCl (10 mL) and brine (10 mL). After drying (MgSO₄) and evaporation of the solvent, 1.18 g of a yellow powder are obtained. When analyzed by NMR, the product was a 82:18 mixture of 7-BOC baccatin III and 7,10-diBOC baccatin III.

## Claims

1. An intermediate for use in the semisynthesis of paclitaxel or a paclitaxel analogue, comprising a compound of the structure: 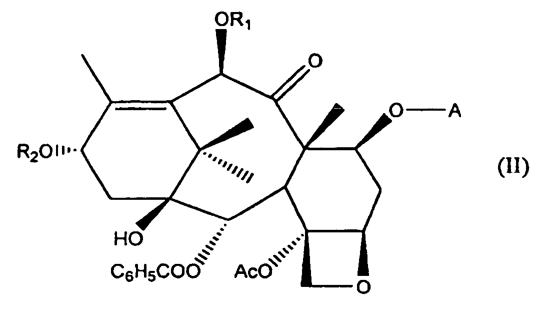 wherein:
A is 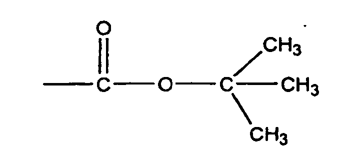
R₁ is a hydroxy-protecting group or a hydrogen atom, and
R₂ is a hydrogen atom.

2. The intermediate of Claim 1 wherein R₁ is A, an acetyl group or a trialkylsilyl group wherein each alkyl group contains 1 to 3 carbon atoms.

3. The intermediate of Claim 1 wherein R₁ represents H.

4. The intermediate of Claim 1 wherein R₁ represents acetyl.

5. An intermediate for use in the semisynthesis of paclitaxel or a paclitaxel analogue, comprising a compound of formula (III): 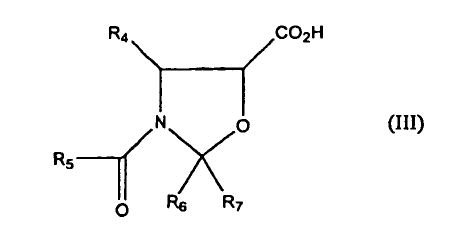 wherein R₄ is an aryl group or a straight or branched chain alkyl or alkenyl group having 1-5 carbon atoms: and R₅ is R₄ or a t-butoxy group, and each of R₆ and R₇ is a halogenated methyl group.

6. The intermediate of Claim 5, wherein R₄ is phenyl, R₅ is phenyl or a t-butoxy group, and each of R₆ and R₇ is a ClCH₂-, BrCH₂- or F₃C-group.

7. A process for producing paclitaxel or docetaxel comprising:
(i) forming an intermediate compound by reacting 10-deacetyl-baccatine III with t-butoxy-pyrocarbonate to obtain 7-t-butoxy-carbonyl-10-deacetyl baccatine III;
(ii) acetylating the 10-position of the 7-t-butoxy-carbonyl-10-deacetyl baccatine III to obtain 7-t-butoxy-carbonyl-baccatine III;
(iii) introducing the group 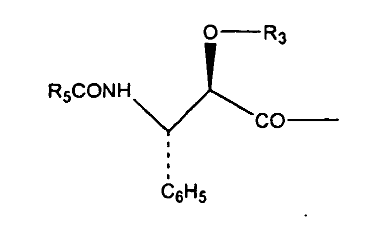 wherein R₃ is a hydrogen atom and R₅ is phenyl or t-butoxy; in a position 13 of 7-t-butoxy-carbonyl baccatine III by reacting the protected baccatine III with an oxazolidine derivative of formula (III): 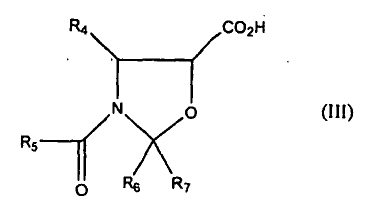 wherein R₄ is phenyl and R₅ is phenyl or a t-butoxy group, and each of R₆ and R₇ is a halogenated methyl group, and selectively removing the A group in mild acidic conditions using a mineral or organic acid.

8. The process of Claim 7 wherein each of R₆ and R₇ is a ClCH₂- or BrCH₂- or F₃C- group.

9. A process according to Claim 7 or Claim 8 wherein an excess of the oxazolidine derivative is used relative to the 7-t-butoxy-carbonyl baccatine III compound.

10. The process of Claim 7 wherein the acetylation is carried out using an acetyl halide or diketene compound.

## Patentansprüche

1. Zwischenprodukt zur Verwendung bei der Halbsynthese von Paclitaxel oder einem Paclitaxelanalogen, umfassend eine Verbindung der Struktur: 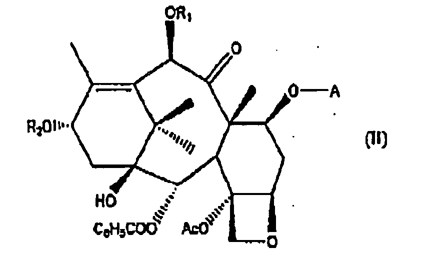 worin:
A 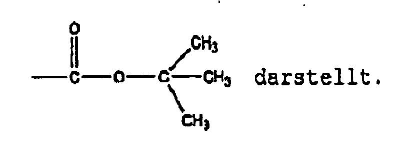
R₁ eine Hydroxyschutzgruppe oder ein Wasserstoffatom darstellt, und
R₂ ein Wasserstoffatom darstellt.

2. Zwischenprodukt nach Anspruch 1, worin R₁ A, eine Acetylgruppe oder eine Trialkylsilylgruppe, worin jede Alkylgruppe 1 bis 3 Kohlenstoffatome enthält, darstellt.

3. Zwischenprodukt nach Anspruch 1, worin R₁ H wiedergibt.

4. Zwischenprodukt nach Anspruch 1, worin R₁ Acetyl wiedergibt.

5. Zwischenprodukt zur Verwendung in der Halbsynthese von Paclitaxel oder einem Paclitaxelanalogen, umfassend eine Verbindung der Formel (III): 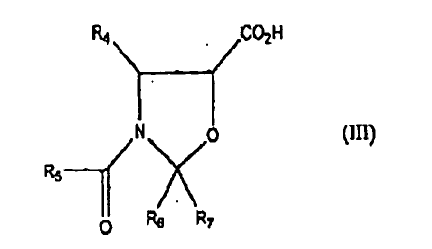 worin R₄ eine Arylgruppe oder eine gerade oder verzweigtkettige Alkyl- oder Alkenylgruppe mit 1 bis 5 Kohlenstoffatomen darstellt und R₅ R₄ oder eine t-Butoxygruppe darstellt und jeder von R₆ und R₇ eine halogenierte Methylgruppe darstellt.

6. Zwischenprodukt nach Anspruch 5, worin R₄ Phenyl darstellt, R₅ Phenyl oder eine t-Butoxygruppe darstellt und jeder von R₆ und R₇ eine ClCH₂-, BrCH₂- oder F₃C-Gruppe darstellt.

7. Verfahren zum Herstellen von Paclitaxel oder Docetaxel, umfassend:
(i) Bilden einer Zwischenproduktverbindung durch Umsetzen von 10-Desacetylbaccatin III mit t-Butoxy-pyrocarbonat zu 7-t-Butoxy-carbonyl-10-desacetylbaccatin III;
(ii) Acetylieren der 10-Position von dem 7-t-Butoxy-carbonyl-10-desacetylbaccatin III zu 7-t-Butoxy-carbonyl-baccatin III;
(iii) Einführen der Gruppe 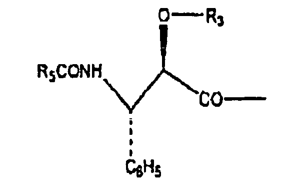 worin R₃ ein Wasserstoffatom darstellt und R₅ Phenyl oder t-Butoxy darstellt; in eine Position 13 von 7-t-Butoxy-carbonylbaccatin III durch Umsetzen des geschützten Baccatins III mit einem Oxazolidinderivat der Formel (III): 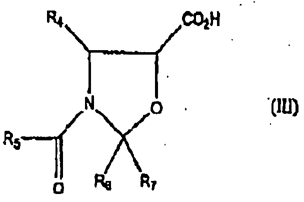 worin R₄ Phenyl darstellt und R₅ Phenyl oder eine t-Butoxygruppe darstellt und jeder von R₆ und R₇ eine halogenierte Methylgruppe darstellt und selektives Entfernen der Gruppe A unter milden sauren Bedingungen unter Anwendung einer Mineral- oder organischen Säure.

8. Verfahren nach Anspruch 7, wobei jeder von R₆ und R₇ eine ClCH₂- oder BrCH₂- oder F₃C-Gruppe darstellt.

9. Verfahren nach Anspruch 7 oder Anspruch 8, wobei, bezogen auf die 7-t-Butoxy-carbonylbaccatin III-Verbindung, ein Überschuss des Oxazolidinderivats verwendet wird.

10. Verfahren nach Anspruch 7, wobei die Acetylierung unter Verwendung eines Acetylhalogenids oder einer Diketenverbindung ausgeführt wird.

## Revendications

1. Intermédiaire utile dans la semisynthèse du paclitaxel ou d'un analogue du paclitaxel, comprenant un composé de structure : 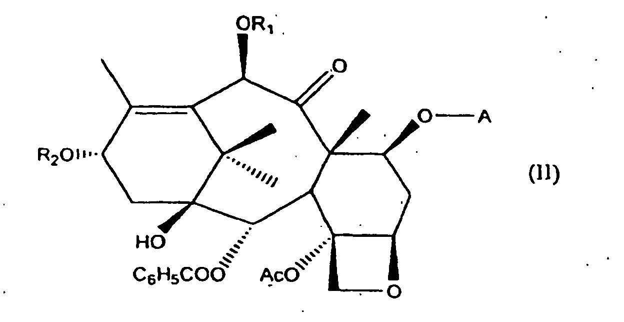 dans laquelle :
A est 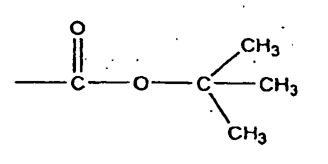
R₁ est un groupe protecteur de la fonction hydroxy ou un atome d'hydrogène, et
R₂ est un atome d'hydrogène.

2. Intermédiaire selon la revendication 1, dans lequel R₁ est A, un groupe acétyle ou un groupe trialkylsilyle dans lequel chaque groupe alkyle contient 1 à 3 atomes de carbone.

3. Intermédiaire selon la revendication 1, dans lequel R₁ représente un atome d'hydrogène.

4. Intermédiaire selon la revendication 1, dans lequel R₁ représente un groupe acétyle.

5. Intermédiaire utile dans la semisynthèse du paclitaxel ou d'un analogue du paclitaxel, comprenant un composé de formule (III) : 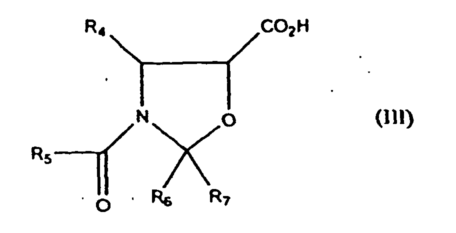 dans laquelle R₄ est un groupe aryle ou un groupe alkyle ou alcényle à chaîne linéaire ou ramifiée ayant 1 à 5 atomes de carbone ; et R₅ est R₄ ou un groupe t-butoxy, et chacun des R₆ et R₇ est un groupe méthyle halogéné.

6. Intermédiaire selon la revendication 5, dans lequel R₄ est un groupe phényle, R₅ est un groupe phényle ou t-butoxy, et chacun des R₆ et R₇ est un groupe ClCH₂-, BrCH₂- ou F₃C-.

7. Procédé pour la production du paclitaxel ou du docétaxel comprenant :
(i) la formation d'un composé intermédiaire par réaction de 10-désacétyl-baccatine III avec du t-butoxy-pyrocarbonate pour obtenir la 7-t-butoxy-carbonyl-10-désacétyl baccatine III ;
(ii) l'acétylation de la position 10 de la 7-t-butoxy-carbonyl-10-désacétyl baccatine III pour obtenir la 7-t-butoxy-carbonyl-baccatine III ;
(iii) l'introduction du groupe 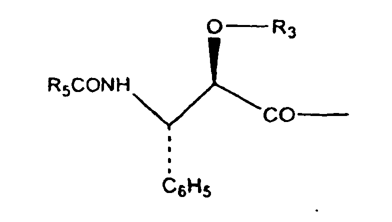 dans lequel R₃ est un atome d'hydrogène et R₅ est un groupe phényle ou t-butoxy ; en position 13 de la 7-t-butoxy-carbonyl baccatine III par réaction de la baccatine III protégée avec un dérivé d'oxazolidine de formule (III) : 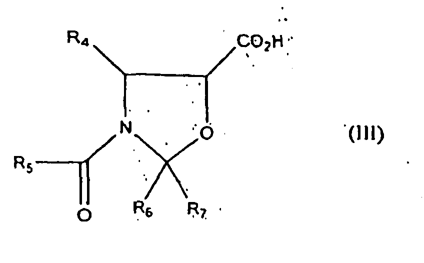 dans laquelle R₄ est un groupe phényle et R₅ est un groupe phényle ou t-butoxy, et chacun des R₆ et R₇ est un groupe méthyle halogéné, et l'élimination sélective du groupe A dans des conditions acides douces avec un acide minéral ou organique.

8. Procédé selon la revendication 7, dans lequel chacun des R₆ et R₇ est un groupe ClCH₂-, BrCH₂- ou F₃C-.

9. Procédé selon la revendication 7 ou la revendication 8, dans lequel un excès du dérivé d'oxazolidine est utilisé par rapport au composé 7-t-butoxy-carbonyl baccatine III.

10. Procédé selon la revendication 7, dans lequel l'acétylation est effectuée avec un halogénure d'acétyle ou un composé dicétène.
